# EUROPEAN PATENT APPLICATION

(11) **EP 1 774 975 A1**
(43) Date of publication of application: **18.04.2007**
(21) Application number: 05757297.6
(22) Date of filing: 09.06.2005
(51) Int. Cl.: A61K 38/43, A61K 38/46, A61K 38/48, A61P 31/00, A61P 31/04

(54) **USE OF HUMAN LYSOZYME IN PREPARATION OF MEDICINE FOR TREATING ACNE**

(30) Priority: 10.06.2004 CN 200410020737
(71) Applicant: An, Mi, Liaoning 116600 (CN)
(72) Inventor: An, Mi, Liaoning 116600 (CN)
(74) Representative: Jannig, Peter
(86) International application number: PCT/CN2005/000821
(87) International publication number: WO 2005/120555

(57) **Abstract**

A new use of human lysozyme is disclosed, especially human lysozyme can be used in the treatment of acne. Human lysozyme can be used for preparing medicine for treating acne induced by staphylococcus aureus, staphylococcus epidermidis, propionibacterium, acarus and drug-resistant bacteria. The medicine can be prepared in the form of spray, drop, ointment and so on.

## Description

### Technological field:

This invention involves the new use of human lysozyme, especially the new use of human lysozyme being used in the treatment of acne.

### Background technology:

The antibiotic has created a lot of medical miracles , make a lot of diseases disappear, for instance pneumonia , meningitis , puerperal fever , septicaemia , tuberculosis ,etc.. Today in the 21 st century, the development drug-resistance bacteria make people shocking. The penicillin-resistant Streptococcus pneumoniae, showed the high susceptibility to penicillin, erythromycin and sulfonamides but now is invulnerability. The resistance rate of Klebsiella pneumoniae to the 16 kinds of high-grade antibiotics such as Cefuroxime, fortum etc. is high to 51.85%-100%. The meticillin sodium-resistant Staphylococcus aureus (MRSA) has no medicine to manage except vancomycin. From the history of bacteria resistance, after a new generation of antibiotic appears, a batch of new drug-resistance bacteria will appear. It takes 10 years or so to develop a kind of new antibiotic, but the production of a new generation of drug-resistance bacteria needs only 2 years. The research speed of the antibiotic can not far catch up with the development speed of the drug-resistance bacteria. At present, a new "super antibiotic" effective to different drug-resistance bacteria was badly needed to be developed for clinical treatment.

### Invention content:

The objective of this invention is to provided a kind of new use of human lysozyme in the treatment of acne induced by Staphylococcus aureus, Staphylococcu epidermidis, Propionibacterium, acarid and drug-resistance bacteria.
Actually, this invention involves the application of human lysozyme in the treatment of acne induced by Staphylococcus aureus, Staphylococcu epidermidis, Propionibacterium, acarid and drug-resistance bacteria.

The human lysozyme stated here are the Recombinant Human Lysozyme expressed by gene-engineering, gene engineering expression Human Lysozyme with the N-terminal modified by (aminoglutaminic acid - 2-aminopropionic acid)₂ or (2-aminopropionic acid - aminoglutaminic acid)₃ , the gene engineering expression or the chemical synthesis pathway mutant Recombinant Human Lysozyme.

There is 300U~3,000,000U/mL Human Lysozyme in the medicine. And the medicine is spray, drop, cream or emulsion. The acaricide SM-650 can also be added in the medicine.

To better understand the essence of the invention. We will illustrate the new use of gene recombinant Human Lysozyme in the pharmaceutical field through the pharmacological pre-test and its results.

Gene recombinant Human Lysozyme was prepared with 200mL medium, phosphonic acid 6mL, magnesium sulfate 3g, potassium sulfate 4g, potassium hydroxide 1g, calcium sulfate 1.5g were added, then added water to 200mL. After autoclaving strain was inoculated, the the inoculate rotation speed of shaker was 250 r/min, culture temperature was 20-35 °C, cultured in the constant temperature bed for 36-48 hour, cultured in the seeding tank then cultured in the fermenter. The culture solution which has completed fermentation was extracted and purified and got the concentrated solution of gene recombinant Human Lysozyme. Determined the protein, tested the activity and conserved the concentrated solution. The concentrated solution of gene recombinant Human Lysozyme with the purity of 95% was dissolved in the water to obtain a nominal concentration of 30000U/mL. Phosphate buffer 20mM (pH7.0), 25% propylene glycol, tween 80 and the acaricide should be prepared. The acaricide SM-650 (the purity was more than 99%), pH:6-8 (commodity), was prepared by dissolving the dry powder in the water to obtain a nominal concentration of 1%.

### Evaluate the antibacterial activity of gene recombinant Human Lysozyme in vitro.

### Materials:

1. The active unit of the concentrated solution of gene recombinant Human Lysozyme (Human Lysozyme HLZ): 30000unit/mL, provided by the Biochemistry Institue of Dalian Qilong.
2. Contral Lysozyme(Contral Lysozyme CLZ): White powder, active unit: 50000 unit/mg, (SIGMA company, USA), Batch No.:L6876.
3. Clarithromycin: potency 948 µ/mg, National Institute for the Control of Pharmaceutical and Biological Products (Beijing, China), Batch No.:
4. Roxithromycin: potency 878 µ/mg, National Institute for the Control of Pharmaceutical and Biological Products (Beijing, China), Batch No.:
5. Amoxicillin: Harbin pharmaceutical factory, Batch No.:
6. Agarose (BIOWEST AGAROSE) :
7. Trihydroxymethyl aminomethane: Chengdu chemical factory, Batch No.:010211

Strain: Pathogenic bacteria isolated from clinical laboratory collected from Sichuan, Beijing, 2002.4-2003.9, assessed using the method of API in our laboratory.

### Strain for quality control:

Staphylococcus aureus ATCC25923
Escherichia coli ATTCC25922
Pseudomonas aeruginosa ATCC27853

### Medium:

1. Tris - HCl buffer solution: 0.1 M Tris 100ml, 0.1M HCl 70ml, High Water 800ml, adjusted pH to 2. 2using HCl solution, added High Water to 1000ml.
2. Tris - HCl agar culture: Added the agarose to the Tris - HCl buffer solution and sterilize at 116°C.
3. M -H Medium: Purchased from National Institute for the Control of Pharmaceutical and Biological Products (Beijing, China). M -H bouillon culture-medium: Heated and dissolved 25g medium in 1000ml distilled water, packed, sterilized by autoclaving for 20min at 116°C. M -H solid medium: Dissolved 25g medium in 1000ml distilled water, sterilized by autoclaving for 20min at 116°C, which would be used in the susceptibility test of Gram-positive bacteria and Gram-negative bacteria.
4. Blood medium: Prepared by adding the 5-10% rabbit blood taken off fibre in the M -H Medium, which would be used in the susceptibility test of enterococci and streptococcic.

### Method:

Test the minimum inhibitory concentration (MIC) of the tested drug against the strains using the double agar dilution method. The tested drug was dissolved in sterile purified water and diluted properly. The drug-containing petri dishes were prepared by spiking 1ml drug conclusion with melted 9ml Tris - HCl agarose solid medium, using agar twofold dilution method and obtained the concentration of 4, 2, 1, 0.5, 0.25, 0.125, 0.06, 0.03 ····· 0.001 mg/ml. The strain solution of 10⁵ CFU/ml was inoculated on the petri dishes using the multipoint inoculator, cultured for 8-10 h at 37 °C, covered the petri dishes using melted 6ml M -H Medium (50 °C) respectively, cultured for 10h at 37 °C. The minimal concentration of the drug-containing petri dishes which had no bacterium growth was the minimal inhibitory concentration (MIC).
Result: (1) The results of the antibacterial activity of Human Lysozyme against Pathogenic bacteria isolated from clinical laboratory were showed in Table 1.
(2) The MIC50 and MIC90 of Human Lysozyme against 379 Pathogenic bacteria isolated from clinical laboratory were showed in Table 2.

According to the results described above: Human Lysozyme has a definite antibacterial activity and also have a very good role against the drug resistant strains. Human Lysozyme a small MW protein, reside in dacryma, phlegm, nasal mucus, blood corpuscle and serum, has bactericidal action against the most Gram-positive bacteria and a few Gram-negative bacteria. The function of gene recombinant Human Lysozyme is mainly cut off the β-1, 4 glucosidic bond between N-acetylglucosamine and N-acetylmuramic acid in the peptidoglycan, destroying the peptidoglycan, causing bacterial lysis.

**Table 1 The antibacterial activity of Human Lysozyme, Chicken lysozyme, Clarithromycin, roxithromycin in vitro**

| MIC | | | | |
|---|---|---|---|---|
| bacteria | HLZ mg/ml(ten thousand µ/ml) | CLZ mg/ml(ten thousand µ/ml) | CLA mg/ml(ten thousand µ/ml) | ROX mg/ml(ten thousand µ/ml) |
| Staphylococcus aureusMRSA02-22 | 0.025(0.03) | 0.008(0.04) | >1 | >1 |
| Staphylococcus aureusMRSA02-23 | 0.025(0.03) | 0.008(0.04) | >1 | >1 |
| Staphylococcus aureusMRSA02-26 | 0.025(0.03) | 0.004(0.02) | >1 | >1 |
| Staphylococcus aureusMRSA02-28 | 0.5(1.5) | 0.016(0.08) | >1 | >1 |
| Staphylococcus aureus02-19-5 | 0.03(0.1) | <0.002(0.001) | >1 | >1 |
| Staphylococcu epidermidis MssE25 | 0.016(0.05) | 0.004(0.02) | <0.001 | <0.001 |
| Staphylococcu epidermidis MRSE02-29 | 0.063(0.2) | 0.5(2.5) | 0.03 | 0.5 |
| Staphylococcu epidermidis MRSE02-5 | <0.001 (0.003) | <0.001 (0.003) | <0.001 | <0.001 |
| Staphylococcu epidermidis MRSE02-6 | <0.001 (0.005) | <0.001 (0.005) | <0.001 | <0.001 |
| Staphylococcu epidermidis MRSE02-20-2 | <0.001 (0.003) | <0.001 (0.005) | > 1 | > 1 |
| Staphylococcu epidermidis MRSE02-20-3 | <0.001 (0.003) | 0.004(0.02) | >1 | >1 |
| Staphylococcu epidermidis MRSE02-20-4 | <0.001 (0.003) | <0.001 (0.005) | > 1 | > 1 |
| Staphylococcu epidermidis MRSE02-20-5 | 0.004(0.012) | <0.001 (0.005) | >1 | >1 |
| Staphylococcu epidermidis MRSE02-20-6 | 0.004(0.012) | <0.001 (0.005) | >1 | >1 |
| Staphylococcu epidermidis MRSE02-20-7 | <0.001 (0.003) | <0.001 (0.005) | 1 | 1 |
| Staphylococcu epidermidis MRSE02-20-8 | <0.001 (0.003) | <0.001 (0.005) | <0.001 | <0.001 |
| Staphylococcu epidermidis MRSE02-20-9 | <0.001 (0.003) | <0.001 (0.005) | <0.001 | <0.001 |
| Staphylococcu epidermidis MRSE02-20-1 | <0.001 (0.003) | <0.001 (0.005) | <0.001 | <0.001 |
| Staphylococcu epidermidis MRSE02-3 | 1(3) | 0.015(0.08) | >1 | >1 |
| Staphylococcu epidermidis MRSE02-4 | 0.004(0.012) | 0.008(0.04) | 0.5 | >1 |
| Staphylococcu epidermidis MRSE02-10 | 0.004(0.012) | 0.25(1.25) | 1 | 1 |
| Staphylococcu epidermidis MRSE02-12 | <0.001 (0.003) | <0.001 (0.005) | 0.008 | 0.125 |
| Staphylococcu epidermidis MRSE02-11 | <0.001 (0.003) | <0.001 (0.005) | <0.001 | <0.001 |
| Staphylococcu epidermidis MRSE02-15 | 0.008(0.024) | 0.002(0.01) | 1 | >1 |
| Staphylococcu epidermidis MRSE02-17 | 0.004(0.012) | <0.001 (0.005) | 0.25 | >1 |
| Staphylococcu epidermidis MRSE02-18 | <0.001 (0.003) | <0.001 (0.005) | <0.001 | <0.001 |
| Staphylococcu epidermidis MRSE02-20 | 0.008(0.024) | <0.001 (0.005) | >1 | >1 |
| Staphylococcu epidermidis MRSE02-21 | 0.016(0.05) | 0.25(1.25) | <0.001 | <0.001 |
| Staphylococcu epidermidis MRSE02-22 | 0.004(0.012) | <0.001 (0.005) | <0.001 | <0.001 |
| Propionibacterium 02-7-4 | 0.008(0.02) | 4(20) | 0.002 | 0.002 |
| Propionibacterium 02-7-5 | 0.008(0.02) | 0.25(0.63) | <0.001 | <0.001 |
| Propionibacterium 02-7-6 | 0.008(0.02) | 0.063(0.31) | 0.008 | 0.25 |
| Propionibacterium 02-7-9 | 0.063(0.2) | 0.125(0.63) | 0.008 | 0.016 |
| Propionibacterium 02-7-7 | 0.125(0.4) | 0.016(0.08) | 0.032 | 0.25 |
| Propionibacterium 03-2-22 | 0.032(0.1) | 0.5(2.5) | 1 | 1 |
| Propionibacterium 03-2-30 | 0.063(0.2) | 4(10) | > 1 | >1 |
| Propionibacterium 03-2-32 | 0.016(0.05) | 0.25(1.25) | 0.008 | 0.5 |
| Propionibacterium 03-2-33 | 0.032(0.1) | 2(10) | >1 | >1 |
| Propionibacterium 03-2-36 | 0.063(0.2) | 4(20) | 0.25 | 0.5 |
| Propionibacterium 03-2-37 | 0.032(0.1) | >4(20) | >1 | 1 |
| Propionibacterium 03-2-39 | 0.032(0.1) | 0.5(2.5) | 0.5 | 1 |
| Escherichia coli 03-2-41 | 0.032(0.1) | 0.5(2.5) | >1 | >1 |
| Escherichia coli 03-2-42 | 0.016(0.05) | 0.5(2.5) | 0.063 | >1 |
| Escherichia coli 03-2-43 | <0.001(0.003) | >4(20) | 0.016 | 0.25 |
| Escherichia coli 03-2-45 | 0.032(0.1) | 2(10) | 0.25 | 0.5 |
| Escherichia coli 03-2-51 | 0.032(0.1) | >4(20) | >1 | >1 |
| Escherichia coli 03-2-52 | 0.032(0.1) | >4(20) | >1 | 0.25 |
| Escherichia coli 03-2-53 | 0.008(0.024) | 4(20) | 0.03 | 0.25 |
| Escherichia coli 03-2-54 | 0.032(0.1) | 1(5) | >1 | 1 |
| Escherichia coli 03-2-56 | 0.032(0.1) | >4(20) | 0.016 | 0.25 |
| Escherichia coli 03-2-57 | <0.001(0.003) | 2(10) | 0.032 | 0.25 |
| Escherichia coli 03-2-58 | <0.001(0.003) | 4(20) | > 1 | 0.5 |
| Escherichia coli 03-2-59 | 0.032(0.1) | 4(20) | 0.125 | 0.25 |
| Serratia marcescens 2-31-8 | 0.008(0.02) | 0.5(0.25) | 0.008 | 0.016 |
| Serratia marcescens 2-31-8 | 0.008(0.02) | 1(5) | 0.008 | 0.016 |
| Serratia marcescens 2-31-8 | 0.008(0.02) | 0.125(0.63) | 0.016 | 0.016 |
| Klebsiella pneumoniae 02-6-14 | 0.25(0.8) | 1(5) | 0.063 | >1 |
| Klebsiella pneumoniae 02-6-18 | 0.5(1.5) | >4(20) | 0.5 | 1 |

| | | | | |
|---|---|---|---|---|
| HLZ: Human Lysozyme CLZ: Chicken lysozyme CLA: Clarithromycin ROX: roxithromycin | | | | |

### Evaluate the activity of killing itch mite of gene recombinant Human Lysozyme plus the acaricide SM-650 in vitro.

### 1. The experiment using the concentrated solution of gene recombinant Human Lysozyme : 300 unit/ml

### Materials:

1. The activity unit of the concentrated solution of gene recombinant Human Lysozyme : 300 unit/ml, provided by the Biochemistry Institue of Dalian Qilong.

2. The acaricide SM-650: white powder, the purity is higher than 99%, pH: 6-8, provided by the Biochemistry Institue of Beijing Naite.

### Acarid for test:

The acarid used in the experiment was collected from the acne vulgaris patients of fourth military medical university.

### Method:

Asses the activity of killing itch mite *in vitro:* The minimal inhibitory concentration (MIC) of Human Lysozyme plus the acaricide SM-650 was assessed using the picture method. Human Lysozyme 300 unit/ml and the acaricide SM-650 1% M1 were dropped on the slide with the acarid specimen. The results of MIC were showed in Table 3.

The results showed that Human Lysozyme plus the acaricide SM-650 has the activity of killing itch mite *in vitro.*

**Table 3.**

| acarid | control | acaricide SM-650 | Human Lysozyme plus the acaricide SM-650 |
|---|---|---|---|
| NO.1 12 acarid | 0 | 51% | 62% |
| NO.2 18 acarid | 0 | 48% | 59% |
| NO.3 16 acarid | 0 | 49% | 61% |
| N0.4 11 acarid | 0 | 50% | 65% |
| NO.5 19 acarid | 0 | 53% | 66% |

### 2. The experiment using the concentrated solution of gene recombinant Human Lysozyme : 3000 unit/ml

### Materials:

1. The activity unit of the concentrated solution of gene recombinant Human Lysozyme : 3000 unit/ml, provided by the Biochemistry Institue of Dalian Qilong.

2. The acaricide SM-650: white powder, the purity is higher than 99%, pH: 6-8, provided by the Biochemistry Institue of Beijing Naite.

### Acarid for test:

The acarid used in the experiment was collected from the acne vulgaris patients of fourth military medical university.

### Method:

Asses the activity of killing itch mite *in vitro:* The minimal inhibitory concentration (MIC) of Human Lysozyme plus the acaricide SM-650 was assessed using the picture method. Human Lysozyme 3000 unit/ml and the acaricide SM-650 1% M1 were dropped on the slide with the acarid specimen. The results of MBC were showed in Table 4.

The results showed that Human Lysozyme plus the acaricide SM-650 has the activity of killing itch mite *in vitro.*

**Table 4.**

| acarid | control | acaricide SM-650 | Human Lysozyme plus the acaricide SM-650 |
|---|---|---|---|
| NO.1 12 acarids | 0 | 73% | 85% |
| NO.2 18 acarids | 0 | 82% | 91% |
| NO.3 16 acarids | 0 | 80% | 94% |
| NO.4 11 acarids | 0 | 76% | 88% |
| NO.5 19 acarids | 0 | 79% | 90% |

### 3. The experiment using the concentrated solution of gene recombinant Human Lysozyme : 30000 unit/ml

### Materials:

1. The activity unit of the concentrated solution of gene recombinant Human Lysozyme : 30000 unit/ml, provided by the Biochemistry Institue of Dalian Qilong.
2. The acaricide SM-650: white powder, the purity is higher than 99%, pH: 6-8, provided by the Biochemistry Institue of Beijing Naite.

### Acarid for test:

The acarid used in the experiment was collected from the acne vulgaris patients of fourth military medical university.

### Method:

Asses the activity of killing itch mite *in vitro:* The minimal inhibitory concentration (MIC) of Human Lysozyme plus the acaricide SM-650 was assessed using the picture method. Human Lysozyme 30000 unit/ml and the acaricide SM-650 1% M1 were dropped on the slide with the acarid specimen. The results of MIC were showed in Table 5.

The results showed that Human Lysozyme plus the acaricide SM-650 has the activity of killing itch mite *in vitro.*

**Table 5.**

| acarid | control | acaricide SM-650 | Human Lysozyme plus the acaricide SM-650 |
|---|---|---|---|
| NO.1 12 acarid | 0 | 100% | 100% |
| N0.2 18 acarid | 0 | 100% | 100% |
| NO.3 16 acarid | 0 | 100% | 100% |
| N0.4 11 acarid | 0 | 100% | 100% |
| NO.5 19 acarid | 0 | 100% | 100% |

The activity of killing itch mite of gene recombinant Human Lysozyme plus the acaricide SM-650 against the acarid collected from five acne vulgaris patients *in vitro* was assesed using the picture method. The results showed that Human Lysozyme plus the acaricide SM-650 has the activity of killing itch mite. The MIC_{Range} of the acaricide SM-650 was 0.2%~2%. The activity unit of Human Lysozyme: 300 unit/ml ~ 30000 ten thousand unit/ml. The gene recombinant Human Lysozyme developed by the Biochemistry Institue of Dalian Qilong showed the same mechanism of action as the other Lysozyme, mainly cut off the β-1, 4 glucosidic bond between N-acetylglucosamine and N-acetylmuramic acid in the peptidoglycan, destroying the peptidoglycan, causing bacterial lysis.

According to the results described above, we can see the advantage of this invention.
(1) This invention has opened up a new application to the medical use new in exploration of Human Lysozyme.
(2) Human Lysozyme is safe and has no adverse effect. There are very good medical prospects.
(3) Human Lysozyme source of this invention is abundant, it is simple to be made to spray, drop, cream or emulsion, easy to use.

### Mode of execution:

Now combining the instance will do further description to this invention:

### Instance one:

After autoclaving strain was inoculated to Gene recombinant Human Lysozyme, the the inoculate rotation speed of shaker was 250 r/min, culture temperature was 20 °C, cultured in the constant temperature bed for 36 hour, cultured in the seeding tank then cultured in the fermenter. The culture solution which has completed fermentation was extracted and purified and got the concentrated solution of protein, lyophilized the solution. Determined the protein, purity, tested the activity and conserved the concentrated solution. The concentrated solution of gene recombinant Human Lysozyme with the purity of 95% being prepared to obtain a nominal concentration of 300U~300 ten thousand U/mL, phosphate buffer 10~20mM (pH6.5~7.5)80%, 5~25% propylene glycol, 0.05% tween 80 were mixed at common temperature to prepare (in the pharmaceutical factory which meet GMP) spray, drop.

### Instance two:

The concentrated solution of gene recombinant Human Lysozyme with the purity of 95% being prepared to obtain a nominal concentration of 300U~300 ten thousand U/mL, phosphate buffer 10mM (pH 6.0)80%, 20% propylene glycol, 6% 2-Pyrrolidone-5-carboxylic acid sodium salt, 0.9% water-soluble azone, 0.03% tween 80, the acaricide SM-650 with the purity >99%, pH: 6-8 (commodity) 1%, were mixed at common temperature to prepare spray, drop.

### Instance three:

The concentrated solution of gene recombinant Human Lysozyme with the purity of 95% being prepared to obtain a nominal concentration of 300U~300 ten thousand U/mL/g, phosphate buffer 20mM (pH 7.0)85%, 20% propylene glycol, carbopol 1%, essence0.3%, the acaricide SM-650 with the purity > 99%, pH: 6-8 (commodity) 1%, were mixed at <70 °C to prepare cream, emulsion.

## Claims

1. The application of human lysozyme in the treatment of acne induced by Staphylococcus aureus, Staphylococcu epidermidis, Propionibacterium, acarid and drug-resistance bacteria.

2. According to the application in the treatment of acne stated in the claim 1, the character is that human lysozyme are the Recombinant Human Lysozyme expressed by gene-engineering, gene engineering expression Human Lysozyme with the N-terminal modified by (aminoglutaminic acid - 2-aminopropionic acid)₂ or (2-aminopropionic acid - aminoglutaminic acid)₃ , the gene engineering expression or the chemical synthesis pathway mutant Recombinant Human Lysozyme.

3. According to the application in the treatment of acne stated in the claim 1, the character is that there is active 300U~3,000,000U/mL Human Lysozyme in the medicine.

4. According to the application in the treatment of acne stated in the claim 1, the character is that the medicine is spray, drop, cream or emulsion.

5. According to the application in the treatment of acne and acarid stated in the claim 1, 2, 3 or 4, the character is that there is the acaricide SM-650 in the medicine.

6. According to the application in the treatment of acne stated in the claim 1, 2, 3 or 4, the character is that the medicine is spray, drop, which is prepared from gene recombinant Human Lysozyme with the purity of 95% being prepared to obtain a nominal concentration of 300U~300 ten thousand U/mL, phosphate buffer 10~20mM (pH6.5~7.5)80%, 5~25% propylene glycol, 0.05% tween 80.

7. According to the application in the treatment of acne and acarid stated in the claim 5, the character is that the medicine is spray, drop, which is prepared from gene recombinant Human Lysozyme with the purity of 95% being prepared to obtain a nominal concentration of 300U~300 ten thousand U/mL, phosphate buffer 10mM (pH 6.0)80%, 20% propylene glycol, 6% 2-Pyrrolidone-5-carboxylic acid sodium salt, 0.9% water-soluble azone, 0.03% tween 80, the acaricide SM-650 with the purity >99%, pH: 6-8 (commodity) 1 %.

8. According to the application in the treatment of acne and acarid stated in the claim 5, the character is that the medicine is cream, emulsion ,which is prepared from gene recombinant Human Lysozyme with the purity of 95% being prepared to obtain a nominal concentration of 300U~300 ten thousand U/mL/g, phosphate buffer 20mM (pH 7.0)85%, 20% propylene glycol, carbopol 1%, essence0.3%, the acaricide SM-650 with the purity > 99%, pH: 6-8 (commodity) 1%.
